# FASCICULE DE BREVET EUROPEEN

(11) **EP 1 668 036 B1**
(45) Date de publication et mention de la délivrance du brevet: **19.01.2011**
(21) Numéro de dépôt: 04816198.8
(22) Date de dépôt: 15.09.2004
(51) Int. Cl.: A61K 39/00

(54) **PROCEDE POUR L' OBTENTION D'ANTICORPS HUMAINS NEUTRALISANT L'ACTIVITE BIOLOGIQUE D'UNE CYTOKINE HUMAINE**
VERFAHREN ZUR HERSTELLUNG HUMANER ANTIKÖRPER, DIE DIE BIOLOGISCHE WIRKUNG EINES HUMANEN CYTOKINS BLOCKIEREN
METHOD FOR THE PRODUCTION OF HUMAN ANTIBODIES BLOCKING THE BIOLOGICAL ACTIVITY OF A HUMAN CYTOKINE

(30) Priorité: 16.09.2003 FR 0350543
(43) Date de publication de la demande: 14.06.2006
(60) Demande divisionnaire: 09163444.4
(73) Titulaire: NEOVACS, 75016 Paris (FR)
(72) Inventeur: BIZZINI, Bernard, F-11000 Carcassonne (FR); ZAGURY, Daniel, F-75007 Paris (FR); LE BUANEC, Hélène, F-75005 Paris (FR)
(74) Mandataire: Catherine, Alain
(86) Numéro de dépôt international: PCT/FR2004/050436
(87) Numéro de publication internationale: WO 2005/028513

(56) Documents cités:
- WO-A2-98/45331
- FR-A- 2 838 444
- ZAGURY ET AL.: "Anti-IFNalpha immunization raises the IFNalpha-neutralizing capacity of serum - an adjuvant to retroviral tritherapy" BIOMED. AND PHARMACOTHER, vol. 53, 1999, pages 90-92, XP002277611 Paris
- ZAGURY D ET AL: "Toward a new generation of vaccines: The anti-cytokine therapeutic vaccines" PROCEEDINGS OF THE NATIONAL ACADEMY OF SCIENCES OF USA, NATIONAL ACADEMY OF SCIENCE. WASHINGTON, US, vol. 98, no. 14, 3 juillet 2001 (2001-07-03), pages 8024-8029, XP002186083 ISSN: 0027-8424
- VALMORI D. ET AL: "Vaccination with NY-ESO-1 protein and CpG in Montanide induces integrated antibody/Th1 responses and CD8 T cells through cross-priming" PNAS, vol. 104, no. 21, 2007, pages 8947-8952,
- BECK J.T. ET AL: 'Alleviation of systemic manifestations of castleman's disease by monoclonal anti-IL6 antibody' THE NEW ENGLAND JOURNAL OF MEDICINE vol. 330, no. 9, 1994, pages 602 - 605
- WILKE-REECE U. ET AL: 'Toll-like receptor agonists influence the magnitude and quality of memory T cell responses after prime-boost immunization in nonhuman primates' JEM vol. 203, no. 5, 2006, pages 1249 - 1258
- MALASPINA A. ET AL: 'appearanceof immature/transitional B cells in HIV-infected individuals with advanced disease: correlation with increased IL-7' PNQS vol. 103, no. 7, 2006, pages 2262 - 2267
- WU Y. ET AL: 'Phase 1 trial of malaria transmission blocking vaccine candidates Pfs and Pvs25 formulated with montanide ISA 51' PLOSONE vol. 3, no. 7, 2008, pages 1 - 9
- GLENNIE M.J. ET AL: 'clinical trials of antibody therapy' IMMUNOLOGY TODAY vol. 21, no. 8, 2000, pages 403 - 410
- DALUM I. ET AL: 'Breaking of B cell tolerance toward a highly conserved self protein' THE JOURNAL OF IMMUNOLOGY vol. 157, 1996, pages 4796 - 4804
- DALUM I. ET AL: 'Therapeutic antibodies elicited by immunisation against TNF alpha' NATURE BIOTECHNOLOGY vol. 17, 1999, pages 666 - 669
- KIM K.J. ET AL: 'The VEGF prteins: identification of biologically relevant regions by neutralizing monoclona antibodies' GROWTH FACTORS vol. 7, 1992, pages 53 - 64, XP000612291

## Description

### DOMAINE DE L'INVENTION

La présente invention se rapporte à des compositions vaccinales.

### ART ANTERIEUR

Les cytokines sont des protéines produites par de nombreuses cellules, comme les lymphocytes, monocytes, cellules dendritiques, mastocytes, fibroblastes, qui exercent des effets autocrines, paracrines et endocrines sur de nombreux tissus. Les cytokines influencent la survie, la prolifération, la différenciation et la migration cellulaire. Les cytokines peuvent être regroupées, assez schématiquement selon leur activité, certaines sont importantes dans l'induction de la mort cellulaire, par un effet direct ou indirect, via la génération de lymphocytes cytotoxiques ou de cellules NK (IFNγ, TNF, IL-2, IL-15). D'autres cytokines participent aux réponses allergiques comme l'IL-3, l'IL-4, l'IL-5, l'IL-9 et l'IL-13. Certaines sont essentielles dans la régulation de la production des anticorps (IL-4, IL-5, IL-6, IL-9 et IL-10, et IL-13) tandis que d'autres exercent une action pro inflammatoire (II-1, TNFα, IFNγ, chemokines) D'autres cytokines encore possèdent des propriétés anti-inflammatoires (TGF-β, IL-4, IL-10). Cette classification ne reflète cependant pas la complexité des interactions entre les cytokines et leurs cibles cellulaires, caractérisées par une redondance et un pléiotropisme important, sans compter les effets agonistes et antagonistes. Ces caractéristiques du système immunitaire et des cytokines en particulier explique que toute interférence avec son fonctionnement, via par exemple un anticorps bloquant une cytokine, a des effets importants *in vivo.*

Jusqu'à présent les stratégies de thérapeutique vaccinale étaient principalement ciblées sur l'agresseur antigénique, que ce soit un microorganisme, une cellule ou un allergène mais ne cherchaient pas à combattre la dérégulation des cytokines. Mais compte tenu du rôle clé des cytokines, impliquées par exemple dans les phénomènes d'échappement aux défenses immunitaire, ou dans les phénomènes d'inflammation, leur inactivation chez des patients afin d'agir sur leur activité ou leur production est un objectif aujourd'hui couramment recherché, d'autant plus que l'organisme ne dispose pas d'antagonistes naturels susceptibles de contrebalancer efficacement ces cytokines. On connaît , par exemple , des anticorps anticytokine, mais ces derniers sont de titre peu élevé car les cellules B qui les produisent sont dormantes. De plus, ces anticorps naturels sont de faible affinité et ne neutralisent pas l'activité de la cytokine correspondante.

Notamment, le phénomène d'échappement aux défenses immunitaires cellulaires de l'hôte par induction de leur paralysie *in situ* est une stratégie utilisée par de nombreux cancers et est nécessaire à leur survie. Initialement l'immunosuppression reste localisée au niveau de la tumeur, puisque l'individu est encore capable de se défendre contre les autres agressions telles que des infections. Cependant à un stade plus tardif, cette immunosuppression peut s'étendre, se généraliser, ainsi que l'attestent la dissémination de métastases et la grande vulnérabilité du cancéreux face aux infections ceci en dehors même des effets dus à la chimiothérapie ou radiothérapie. En résumé, cet échappement au contrôle du système immunitaire est dû à une paralysie du système immunitaire (immunosuppression), qui l'empêche de fonctionner normalement. Cette immunosuppression met en jeu des facteurs paralysants, y compris des cytokines, produits par les cellules cancéreuses ou leur environnement. La paralysie locale des cellules du système immunitaire ou immunosuppression représente donc une arme majeure des cellules cancéreuses qui leur permet d'échapper au système immunitaire de l'hôte.

Le phénomène d'échappement aux défenses immunitaires cellulaires de l'hôte par induction de leur paralysie in situ est une stratégie également utilisée par le VIH. L'immunosuppression généralisée observée dans le cas du SIDA se caractérise notamment par surproduction d'IFNα par les cellules présentant l'antigène et particulièrement les cellules dendritiques de type 2 (DC2). Les activités immunosuppressives de l'IFNα sont dues à sa capacité d'induire la production de la cytokine IL-10 par les cellules T régulatrices (anciennement T suppressives).

Une première stratégie d'inhibition de l'activité biologique des cytokines consiste à injecter une construction immunogène à un patient. A titre d'exemple, afin de provoquer la reconnaissance d'un antigène d'intérêt, par exemple une cytokine, par les cellules B, on a réalisé diverses constructions immunogènes dans l'état de la technique. Une forme de ces constructions immunogènes consiste en un couplage covalent de l'antigène d'intérêt sur une molécule porteuse, la molécule porteuse apportant des structures reconnues par les lymphocytes T auxiliaires (cellules « T helper »), en association avec des molécules de classe Il du Complexe Majeur d'Histocompatibilité (CMH), et qui activent les lymphocytes T auxiliaires qui produisent alors diverses cytokines, dont l'IL-2, lesquelles cytokines vont à leur tour activer les clones de cellules B spécifiques de l'antigène d'intérêt. Les cellules B spécifiques de l'antigène d'intérêt, une fois activées, vont se multiplier et produire des anticorps spécifiques de l'antigène d'intérêt, ce qui est l'objectif recherché. En général, ce type de constructions immunogènes consiste en des produits du couplage chimique covalent entre l'antigène d'intérêt et la molécule porteuse, lesquels, après une étape de purification et élimination des produits non couplés, sont des produits finaux de structure chimique bien définie.

Néanmoins de telles constructions immunogènes ont des inconvénients, parmi lesquels on peut citer le délai important entre l'injection desdites constructions, et l'apparition d'une réponse immune dirigée contre la cytokine ciblée. De plus, il est parfois impossible de vacciner efficacement des patients avec des constructions immunogènes anti-cytokines, lorsque lesdites cytokines altèrent justement l'intensité ou la qualité de la réponse immune. En effet, dans le cadre par exemple du cancer ou du SIDA, où une immunosuppression de grande ampleur est observée, l'administration d'une composition immunogène doit intervenir, pour être efficace, à un stade précoce de la maladie, en particulier avant que l'immunosuppression ne soit trop importante.

Il existe donc un besoin dans l'état de la technique pour des anticorps anti-cytokines qui permettent de traiter des patients présentant une dépression du système immunitaire. Ces anticorps agissent directement sur les cytokines impliquée dans l'immunosuppression ou encore dans le processus inflammatoire. Un tel traitement par des anticorps anti-cytokines permet de rétablir au moins temporairement la réponse immunitaire et peut alors servir de préalable à l'administration de compositions immunogènes.

Dans l'état de la technique on connaît déjà des anticorps anti-cytokines. On peut citer à titre d'exemple la demande de brevet EP1285930 et la demande de brevet US6,509,015 correspondante décrivant des anticorps humains, et en particulier des anticorps recombinants humains, qui se lient de manière spécifique au TNFα, une cytokine produite dans de nombreux types cellulaires, et connue pour son rôle dans les maladies autoimmunes, les infections, ou les rejets de greffe.

On peut également citer la demande internationale WO 00/56772 décrivant des anticorps humains, de préférence recombinants, qui se lient spécifiquement à l'IL-2 humaine, et neutralisent son activité *in vivo* et *in vitro.*

On peut aussi citer les travaux de Zagury et al. qui décrivent l'obtention, chez des patients immunodéficients asymptomatiques infectés par le virus VIH, d'anticorps neutralisants dirigés contre l'interféron α, par administration chez ces patients de molécules d'interféron α inactivé (voir Zagury et al., 1999, Biorned & Pharmcother, Vol. 53: 90-92).

On peut enfin citer la demande de brevet US 2003/0099647 décrivant des agents de liaison à l'interféron γ et en particulier des domaines de liaison à des anticorps et à des antigènes qui se lient sélectivement à l'IFNγ et qui peuvent être utilisés pour prévenir ou traiter des maladies inflammatoires ou autoimmunes telles que l'arthrite rhumatoïde ou le lupus érythremateux.

Compte tenu du rôle clé des cytokines rappelé ci-dessus, il existe un besoin permanent pour de nouvelles compositions vaccinales capable d'induire des anticorps capable de se lier à des cytokines d'intérêt et de neutraliser au moins partiellement leur activité biologique. Il est important que de telles compositions soient peu coûteuses, simples à préparer et qui puissent être synthétisées de manière reproductible.

### SOMMAIRE DE L'INVENTION

La présente invention foumit des compositions vaccinales comprenant à titre de principe actif, un produit immunogene sable ainsi que leus utilisations, tel que cela est défini dans les revendications.

### DESCRIPTION DETAILLEE DE L'INVENTION

On a montré selon l'invention que de nouveaux anticorps humains à haute capacité de neutralisation de l'activité d'une cytokine humaine, pouvaient être produits par immunisation d'un homme à l'encontre de ladite cytokine, sans nécessiter d'étapes complexes de criblage cellulaire et de purification des procédés de l'art antérieur. Il est utile de préciser, pour plus de clarté, qu'un individu non immunisé à l'encontre d'une cytokine, ne possède pas, naturellement, d'anticorps neutralisant l'activité de ladite cytokine.

De tels anticorps anti-cytokines sont utiles dans de multiples contextes pathologiques dans lesquels ces cytokines sont impliquées. On peut citer à titre d'exemple, les infections, les maladies autoimmunes, les rejets de greffe, et en général toutes les maladies liées à une réaction inflammatoire comme les allergies. Les anticorps comprenant sont utiles pour lutter contre ce type de désordre et de surcroît, sont faciles à obtenir.

L'invention décrit un procédé à haut rendement pour l'obtention d'anticorps humains neutralisant l'activité biologique d'une cytokine humaine choisie parmi le VEGF, l'IFNα, l'IL-4, le TNFα, et le TGFβ, comprenant au moins une étape (a) au cours de laquelle on purifie :
(i) les immunoglobulines contenues dans le sérum d'un individu, ou
(ii) les immunoglobulines produites par des cellules B d'un individu,
ledit individu étant préalablement immunisé à l'encontre de ladite cytokine, avec un produit immunogène tel que décrit dans la demande de brevet Français n°FR0211455 déposée le 16 septembre 2002. Un tel produit est un produit immunogène stable comprenant des hétérocomplexes immunogènes protéiques constitués d'associations entre (i) des molécules de protéines antigéniques, choisies parmi VEGF, l'IFNα, l'IL-4, le TNFα, et le TGFβ et (ii) des molécules protéiques porteuses dans lesquels moins de 40 pour cent des protéines antigéniques (i) sont liées avec les molécules protéiques porteuses (ii) par une liaison covalente.

Le VEGF (Vascular Endothelial Growth Factor), de même que l'IL-4 (Interleukine 4), l'IFNα (interféron α), le TNFα (Tumor Necrosis Factor α) et le TGFβ (Transforming Growth Factor β), seront parfois désignés sous le terme générique « cytokine d'intérêt » dans la suite de la description.

De manière préférée, les anticorps consistent en des anticorps « neutralisants » ou « bloquants ». Un anticorps « neutralisant » ou un anticorps « bloquant » est défini, selon l'invention, comme un anticorps dont la fixation sur la cytokine cible bloque l'activité biologique de cette cytokine, ce qui est l'objectif principal, recherché par l'invention, lorsque la cytokine à l'encontre de laquelle les anticorps sont dirigés possède une activité biologique délétère pour l'organisme.

L'obtention préalable d'une immunisation de l'individu à l'encontre de la cytokine d'intérêt, est détectée, comme illustré dans les exemples, notamment par un titre élevé d'anticorps anti-cytokine dans le sérum des individus immunisés. On a observé que des anticorps naturels humais neutralisant l'activité délétère d'une cytokine d'intérêt étaient obtenus chez tous les individus immunisés.

Sans vouloir être lié par une quelconque théorie, les inventeurs pensent que les anticorps naturels humains anti-cytokine qui sont décrits, sont des anticorps de haute affinité. En d'autres termes, selon les inventeurs, les complexes formés entre lesdits anticorps et la cytokine à l'encontre de laquelle ils sont dirigés, ne se dissocient pas ou très difficilement, ce qui provoque une réduction drastique de la concentration circulante de cytokine libre chez un individu auquel on a administré les anticorps définis dans la présente description.

De manière tout à fait préférée, le sérum humain ou les cellules B humaines produisant ces anticorps proviennent d'un individu immunisé avec un produit immunogène tel que décrit dans la demande de brevet Français n°FR0211455 déposée le 16 septembre 2002. Un tel produit est un produit immunogène stable comprenant des hétérocomplexes immunogènes protéiques constitués d'associations entre (i) des molécules de protéines antigéniques, choisies parmi l'IFNα, l'IL-4, et le TNFα, et (ii) des molécules protéiques porteuses dans lesquels moins de 40 pour cent des protéines antigéniques (i) sont liées avec les molécules protéiques porteuses (ii) par une liaison covalente.

Par « protéine antigénique », on entend une cytokine choisie parmi l'IFNα, l'IL-L et la TNFα, au besoin inactivée chimiquement ou un fragment de ladite cytokine d'au moins 10 acides aminés de longueur, susceptible d'être reconnu spécifiquement par les récepteurs pour l'antigène exprimés par les lymphocytes B d'un organisme hôte, humain ou animal, particulièrement tout mammifère, laquelle protéine antigénique, une fois incluse dans un produit immunogène, stimule la production d'anticorps reconnaissant ladite cytokine.

La protéine d'intérêt antigénique peut aussi consister en un homo-oligomère ou homo-polymere de ladite cytokine native ou encore en un homo-oligomère ou homo-polymère d'un fragment peptidique de cytokine native. La protéine d'intérêt antigénique peut aussi consister en un hétéro-oligomère ou en un hétéro-polymère comprenant une combinaison de plusieurs fragments peptidiques distincts initialement inclus dans la cytokine native.

Par « molécule protéique porteuse », incluse dans le produit immunogène, on entend toute protéine ou peptide d'au moins 15 acides aminés de longueur, quelle que soit sa séquence en acides aminés, et qui, en s'associant de manière partiellement covalente aux molécules d'antigène d'intérêt pour former les hétérocomplexes protéiques constitutifs du produit immunogène, permet la présentation d'un grand nombre de molécules d'antigène d'intérêt aux lymphocytes B. De préférence, les molécules protéiques porteuses seront du type KLH (Keyhole Limpet Hemocyanin).

De préférence, le produit immunogène comprend en outre des oligodésoxynucléotides CpG à titre d'adjuvant qui permettent d'augmenter la production d'anticorps neutralisant l'activité de la cytokine d'intérêt. L'utilisation d'oligonucléotides CpG comme adjuvant d'une composition immunogène, pour augmenter la réponse immunitaire, est décrite dans les documents suivants : McCluskie MJ et al. 2000 ; Gallichan WS, et al., 2001 ; et Eastcott JW et al., 2001.

A titre d'exemple de produit immunogène stable comprenant des hétérocomplexes efficaces pour induire une immunisation qui constitue le principe actif de la composition vaccinale de l'invention, on peut citer l'association entre la molécule protéique porteuse KLH (Keyhole limpet hemocyanin) et des molécules d'IFNα humain, héterocomplexe ci-après désigné sous le terme IFNα-KLH. Les produits immunogènes préférés sont choisis parmi les produits immunogènes comprenant les hétérocomplexes suivants, dans lesquels les protéines antigéniques (i), d'une part et la molécule porteuse protéique (ii), d'autre part, sont respectivement :
a> (i) interféron alpha et (ii) KLH ;
b> (i) IL-4 et (ii) KLH ;
c> (i) TNFα et (ii) KLH ; et

Le pourcentage de molécules protéiques porteuses et de protéines antigéniques d'intérêt liées entre elles par des liaisons covalentes dans un produit immunogène de l'invention peut être aisément vérifié par l'homme du métier.

Par exemple, la détermination du pourcentage de molécules d'antigène d'intérêt liées aux molécules de protéine porteuse par une liaison covalente dans un produit immunogène de l'invention peut être réalisée suivant le protocole décrit dans l'exemple 10.

A titre illustratif, le produit immunogène comprenant des hétérocomplexes entre la molécule protéique porteuse KLH (Keyhole limpet hemocyanin) et des molécules d'interféron alpha humain, seulement 3 % des molécules d'interféron alpha sont liées de manière covalente à la molécule protéique porteuse KLH.

Le produit immunogène comprenant des hétérocomplexes immunogènes tels que définis ci-dessus qui constitue le principe actif d'une composition vaccinale selon l'invention, peut être préparé selon les étapes suivantes :
a) incuber les protéines antigéniques (i) et la molécule porteuse (ii) dans un rapport molaire (i) : (ii) de 5::1 à 50::1, en présence d'un agent chimique de liaison;
b)récupérer le produit immunogène comprenant les hétérocomplexes immunogènes préparé à l'étape a);

De préférence, l'agent chimique de liaison est le glutaraldéhyde.

De manière tout à fait préférée, le procédé est en outre **caractérisé en ce que** l'étape a) est suivie d'une étape de stabilisation du produit comprenant les hétérocomplexes immunogènes par le formaldéhyde, préalablement à l'étape b) de récupération des hétérocomplexes.

De préférence, lorsque le glutaraldéhyde est utilisé comme agent chimique de liaison, il est présent dans le milieu réactionnel de couplage à une concentration finale comprise entre 0,002 M et 0,03 M, de préférence à la concentration finale de 0,026M.

La réaction de couplage avec le glutaraldéhyde est avantageusement réalisée, pendant 20 minutes à 60 minutes de préférence 30 minutes, à température allant de 20 à 25°C.

Après l'étape de couplage, le glutaraldéhyde en excès est éliminé, par exemple par dialyse à l'aide d'une membrane de dialyse ayant un seuil de coupure de 3 kDa. L'étape de dialyse est avantageusement réalisée à 4°C, dans un tampon ajusté à pH 7,6.

Pour stabiliser le produit comprenant les hétérocomplexes protéiques préparé à l'étape a), ledit produit peut être traité en solution par le formaldéhyde, par exemple par du formaldéhyde à la concentration finale de 3 mM. La réaction de stabilisation a avantageusement une durée comprise entre 12 et 48 heures, de préférence entre 20 et 30 heures et est de manière tout à fait préférée d'une durée de 24 heures. La réaction de stabilisation par le formaldéhyde est avantageusement stoppée par addition de glycine, de préférence à la concentration de 0,1 M, pendant 1 heure et à une température comprise entre 20 et 25°C.

L'adjonction d'oligodésoxynucléotides CpG intervient de préférence après l'étape d'élimination de l'excès de glutaraldéhyde.

La préparation d'une composition vaccinale comprenant un produit immunogène comprenant des hétérocomplexes immunogènes tels que définis ci-dessus est en outre illustrée dans les exemples 1, 2, 3 et 4.

Typiquement, les anticorps naturels humains neutralisants qui sont décrits neutralisent au moins 50% du maximum de l'activité biologique de la cytokine d'intérêt *in vitro.*

Par exemple, on a montré que l'inhibition *in vitro* d'au moins 50% du maximum de l'activité biologique d'une cytokine d'intérêt est couramment obtenue avec une quantité d'anticorps naturels humains neutralisants allant de 10⁻³ µg à 10⁻²µg.

Le maximum d'activité biologique de la cytokine d'intérêt *in vitro* est préférentiellement induit par, respectivement :
(i) la quantité minimale d'IFNα provoquant le maximum d'inhibition de la lyse des cellules de la lignée MDBK par le virus de la stomatite vésiculaire (VSV) ;
(ii) la quantité minimale d'IL-4 provoquant le maximum de prolifération de la lignée cellulaire TF-1 dépendante de l'IL-4.
(iii) la quantité minimale de TNFα provoquant le maximum de prolifération de la lignée cellulaire L929 dépendante du TNFα.

Les tests d'activité biologique de l'IFNα humain, de l'IL-4, et du TNFα, peuvent être conduits notamment selon le protocole décrit respectivement dans les exemples 6, 7, et 8.

De manière préférée, le procéde décrit comprend une étape (b) de purification des immunoglobulines d'isotype G à partir de la fraction d'immunoblobulines obtenues à l'issue de l'étape (a).

Le procédé décrit est particulièrement avantageux car il permet d'obtenir un haut niveau de réponse anticorps, et une forte proportion de cellules B circulantes produisant des anticorps neutralisant l'activité de la cytokine d'intérêt. La population de lymphocytes B isolée peut être directement utilisée, par exemple en fusionnant ces lymphotyctes B avec des cellules de myélome pour produire les anticorps naturels humains anti cytokine, ce qui évite les complexes de purification. Le procédé décrit permet par exemple d'éviter d'avoir recours à des méthodes de purification des cellules B telles que le panning, le filtrage à travers des colonnes contenant des anticorps couplés au nylon ou encore le triage par cytométrie de flux (FACS®).

L'obtention des cellules B à partir de l'individu immunisé est simple. Une première méthode consiste à isoler les lymphocytes B à partir du sang périphérique, par centrifugation dans un gradient de densité constitué d'un mélange de polymères polysaccharidiques (Ficoll®) et de métrizamide, un composé iodé dense. On obtient à l'interface une population de lymphocytes B séparée des globules rouges et de la plupart des polynucléaires.

Une autre méthode d'obtention des cellules B à partir de l'individu immunisé, consiste à isoler les lymphocytes B à partir des tissus et en particulier à partir des organes lymphoïdes comme la rate, le thymus, la moelle osseuse, les ganglions ou les tissus lymphoïdes associés aux muqueuses, comme les amygdales palatines.

Le procédé décrit peut éventuellement comprendre une étape ultérieure supplémentaire d'obtention d'anticorps polyclonaux monospécifiques neutralisant l'activité biologique de la cytokine.

Le procédé décrit peut aussi comprendre une étape ultérieure supplémentaire d'obtention de fragments F(ab) ou F(ab)'₂ neutralisant l'activité biologique de la cytokine.

Ledit procédé peut aussi comprendre les étapes additionnelles suivantes :
(i) fusionner les cellules B obtenues à l'issue de l'étape (a) avec des cellules d'un myélome, afin d'obtenir une lignée d'hybridomes, et
(ii) récupérer les anticorps produits par les hybridomes.

L'ensemble de ces étapes sera décrit plus en détail dans la suite de la description concernant des compositions pharmaceutiques. On décrit aussi une composition pharmaceutique **caractérisée en ce qu'**elle comprend, à titre de principe actif, des anticorps naturels humains d'isotype Ig4, neutralisant l'activité d'une cytokine humaine choisie parmi le VEGF, l'IFNα l'IL-4, le TNFα, et le TGFβ, lesdits anticorps neutralisants inhibant au moins 50% du maximum d'activité biologique induite par ladite cytokine *in vitro.*

Comme il ressort de la présente description, les anticorps naturels humains contenus dans la composition pharmaceutique telle que définie ci-dessus sont obtenus à partir du sérum ou des cellules B provenant d'un individu préalablement immunisé à l'encontre de ladite cytokine d'intérêt à l'aide de la composition vaccinale de l'invention.

Typiquement, ces anticorps naturels humains inhibent *in vitro* au moins 50% de l'activité biologique induire par une quantité allant de 0,006 ng à 0,5 ng de ladite cytokine d'intérêt.

Par exemple, on a montré que l'inhibition *in vitro* d'au moins 50% du maximum de l'acivité biologique de ladite cytokine d'intérêt, par exemple lorsque cette cytokine est utilisée dans les quantités spécifiées ci-dessus, est couramment obtenue avec une quantité d'anticorps naturels humains neutralisants anti-cytokine allant de 10⁻³ µg à 10⁻² µg.

De préférence lesdits anticorps naturels humains neutralisants sont obtenus à partir du sérum ou de cellules B d'un individu immunisé avec la composition vaccinale selon l'invention qui comprend, à titre de principe actif, un produit immunogène stable comprenant des hétérocomplexes immunogènes protéiques constitués d'associations entre (i) des molécules de protéines antigéniques, respectivement choisies parmi le l'IFNα, l'IL-4, et le TNFα, et (ii) des molécules protéiques porteuses dans lesquels moins de 40 pour cent des protéines antigéniques (i) sont liées avec les molécules protéiques porteuses (ii) par une liaison covalente.

Par « anticorps naturels », on entend exclusivement les anticorps tels qu'ils sont produits naturellement par l'individu immunisé spécifiquement à l'encontre de la cytokine d'intérêt, lesdits anticorps naturels étant contenus dans le sérum du sang de cet individu, ou ces anticorps naturels étant produits, après immunisation, par les cellules B activées de cet individu.

La définition des hétérocomplexes, leur obtention, la détermination du pourcentage de liaison des protéines antigéniques avec les protéines porteuses, peuvent être conduits selon des techniques identiques à celles qui ont été décrites en ce qui concerne le procédé de l'invention.

De manière préférée, le maximum d'activité biologique de ladite cytokine *in vitro* est induit par, respectivement :
(i) 0,5 ng de VEGF
(ii) 0,006 ng d'IFNα
(iii) 0,5 ng d'IL-4.

Les anticorps naturels humains neutralisants anti-cytokine spécifiés dans la présente description sont des anticorps polyclonaux ou monoclonaux.

De préférence, les anticorps polyclonaux sont choisis parmi :
(i) une fraction totale d'anticorps purifiés à partir du sérum d'un homme immunisé à l'encontre de ladite cytokine humaine ;
(ii) une fraction purifiée d'anticorps polyclonaux monospécifiques neutralisant l'activité de ladite cytokine ;
(iii) les fragments Fab ou F(ab)'2 préparés à partir des anticorps polyclonaux (i) et (ii) ci-dessus.

L'obtention d'une fraction totale d'anticorps (i) purifiés à partir du sérum d'un homme immunisé à l'encontre de ladite cytokine humaine comprend de préférence les étapes suivantes :
a) l'injection d'une composition immunogène, de préférence telle que décrite ci-dessus, à un homme,
b) récupération de l'immunsérum de cet homme, contenant les anticorps neutralisant l'activité de l'antigène administré,
c) La purification d'une fraction d'anticorps à partir de l'immunsérum.

Chacune de ces étapes est décrite plus en détail ci-dessous. La récupération de l'immunsérum est réalisée d'une manière connue de l'homme du métier, par exemple par séparation du sérum par centrifugation d'un échantillon de sang total. La purification d'une fraction d'anticorps à partir de l'immunsérum d'un patient immunisé par exemple par chromatographie d'affinité, précipitation au sulfate d'ammonium, chromatographie échangeuse d'ions, filtration sur gel, chromatographie sur colonne de Protéine A / G, chromatographie d'affinité, ou chromatographie d'immunoaffinité.

Une étape supplémentaire de purification des anticorps, particulièrement bien adaptée est décrite dans la demande de brevet EP662480. Cette méthode permet de supprimer les anticorps anti-protéine porteuse au sein d'un mélange d'anticorps, et en particulier au sein de l'immunsérum obtenu à l'étape (b).

Une fraction purifiée d'anticorps polyclonaux monospécifiques (ii) neutralisant l'activité de la cytokine d'intérêt peut être obtenue en réalisant une chromoatographie d'affinité à partir d'une fraction totale d'anticorps (i) purifiés à partir du immunsérum d'un homme immunisé à l'encontre de ladite cytokine humaine, en fixant un motif antigénique de la cytokine d'intérêt sur la colonne.

La purification des fragments F(ab')₂ à partir des anticorps polyclonaux décrits ci-dessus ou à partir de l'immunsérum ou du plasma sanguin peut être conduite selon la méthode décrite dans la demande de brevet US2002/0164327, comprenant une étape de digestion du plasma sanguin ou du sérum par la pepsine et des étapes de séparation et de purification jusqu'à obtention de fragments F(ab')₂, dépourvus d'albumine, d'anticorps complets, et substantiellement dépourvus de substances pyrogènes.

L'isolement de fractions F(ab) et F(ab')₂ permet d'obtenir des avantages spécifiques, comme le fait de se fixer aux cytokines d'intérêt sans pour autant interagir avec d'autres molécules effectrices du système immunitaire.

Les fragments F(ab), peuvent être obtenus par une méthode similaire, consistant à digérer l'immunsérum, le plasma ou les fractions purifiées d'anticorps polyclonaux (i), (ii) et (iii) provenant d'un homme immunisé contre une cytokine d'intérêt, par la papaïne.

Alternativement, les anticorps sont monoclonaux et choisis parmi :
(i) des anticorps produits par des cellules issues de la fusion cellulaire entre (a) des cellules B d'un homme immunisé à l'encontre de ladite cytokine humaine et (b) des cellules d'une lignée cellulaire productrice d'anticorps, telle que des cellules d'un myélome ;
(ii) des anticorps produits par des cellules transfectées ou transformée avec un ADN codant une immunoglobuline, ledit ADN étant préalablement isolé à partir de l'ADN d'une cellule B d'un homme immunisé à l'encontre de ladite cytokine humaine ; selon une caractéristique particulière, ladite immunoglobuline reproduit les anticorps induits par l'immunisation et qui sont présents dans le sérum des patients immunisés.
(iii) les fragments Fab ou F(ab)'2 préparés à partir des anticorps polyclonaux (i) et (ii) ci-dessus ;
(iv) des fragments ScFv .

Les anticorps monoclonaux (i) peuvent être obtenus de la manière suivante :

La première étape consiste à isoler les lymphocytes B à partir d'un homme immunisé à l'encontre d'une cytokine humaine. Une première méthode consiste à isoler les lymphocytes B à partir du sang périphérique, par centrifugation dans un gradient de densité constitué d'un mélange de polymères polysaccharidiques (Ficoll®) et de métrizamide, un composé iodé dense. On obtient à l'interface une population de cellules mononucléaires séparée des globules rouges et de la plupart des polynucléaires. La population de lymphocytes B peut ensuite être fusionnées avec des cellules de myélome ou subir une étape de purification supplémentaire facultative, par la méthode de « panning » c'est à dire par fixation à un support recouvert d'anticorps permettant une adhérence spécifique des lymphocytes B. Les cellules peuvent aussi être filtrées à travers des colonnes contenant des anticorps couplés au nylon qui recouvre de la laine d'acier, ce qui permet l'élution de différentes population cellulaires, dont les lymphocytes B. Ces techniques peuvent fournir un préalable à un triage par FACS® qui fournit des populations cellulaires hautement purifiées.

Une autre méthode consiste à isoler les lymphocytes B à partir des tissus et en particulier à partir des organes lymphoïdes comme la rate, le thymus, la moelle osseuse, les ganglions, ou les tissus lymphoïdes associés aux muqueuses, comme les amygdales palatines. En cas de réponse immune locale, les lymphocytes peuvent être isolés du site même de la réaction.

Les lymphocytes ainsi isolés sont ensuite fusionnés avec des cellules de myélome, les cellules hybrides ou hybridomes sont ensuite sélectionnés en fonction de leur affinité pour la cytokine d'intérêt et les hybridomes produisant un anticorps de spécificité voulue sont ensuite identifiés et clonés par repiquage. Les anticorps produits par de tels hybridomes, produisant les anticorps de haute affinité envers la cytokine d'intérêt constituent la fraction d'anticorps monoclonaux (i).

Un type particulier de lymphocyte B peut aussi être isolé par culture en dilution limite, et sélectionné en fonction de l'aptitude des anticorps qu'il produit à se lier à la cytokine d'intérêt, avant d'être fusionné avec des cellules de myélome.

L'ADN provenant d'un lymphocyte B ou d'un hybridome sélectionné pour sa capacité à produire des anticorps de haute affinité pour une cytokine peut ensuite être isolé, et les séquences d'ADN codant pour l'anticorps peuvent être amplifiées selon des techniques classiques en biologie moléculaire. L'ADN ainsi amplifié est ensuite inséré dans des cellules permettant l'expression des anticorps du lymphocyte B sélectionné. Les anticorps ainsi produits constituent la fraction (ii).

Les fragments F(ab) et F(ab')₂ (iii) peuvent être isolés selon une méthode enzymatique identique à celle décrite ci-dessus pour les anticorps polyclonaux.

Les fragments scFv (iv) ou « Single chain Variable Fragment » peuvent être synthétisés de la manière suivante :

Les séquences d'ADN codant pour le domaine variable d'une chaîne lourde et le domaine variable d'une chaîne légère sont sélectionées à partir de l'ADN d'un lymphocyte B ou d'un hybridome tel que décrit ci-dessus. Cette sélection peut intervenir en choisissant des amorces spécifiques, selon des techniques classiques de biologie moléculaire. De telles séquences d'ADN sont ensuite clonées dans un vecteur d'expression, avec une séquence codant un peptide synthétique dont le rôle est de lier les domaines variables.

Le vecteur d'expression est ensuite inséré dans une cellule hôte. Des exemples de vecteurs d'expression et de cellules hôtes adaptées à l'expression d'anticorps et de fragments d'anticorps sont fournis ci-après. On peut citer à titre d'exemple de cellules hôte, bien adaptées à la synthèse de fragment scFv, les cellules L-form de *Proteus mirabilis* comme cela est décrit par Rippmann et al. (1998).

La synthèse de fragments scFv permet d'obtenir des avantages spécifiques du fait de la petite taille de ces fragments, qui leur permet de diffuser facilement dans les tissus.

Il est également possible de synthétiser des fragments Fab et F(ab)'₂, selon la méthode décrite ci-dessus en ce qui concerne les fragments scFv.

Bien que cela ne constitue pas un mode de réalisation préféré de l'invention, les anticorps naturels humains neutralisants anti-cytokine spécifiés dans la présente description peuvent être recombinants. Dans ce cas, les fragments d'ADN issus d'un lymphocyte B ou d'un hybridome sélectionné selon la méthode décrite ci-dessus, subissent des modifications. En particulier, L'ADN codant pour les domaines variables de l'anticorps, et en particulier pour les domaines variables des chaînes lourdes (VH) et des chaînes légères (VL) peut être muté de manière aléatoire, pour augmenter l'affinité des fragments vis-à-vis d'une cytokine d'intérêt. Les domaines variables peuvent aussi être mutés dans la région CDR3 selon un procédé analogue à la mutation somatique responsable de la maturation de l'affinité des anticorps durant la réponse immune naturelle. La maturation de l'affinité *in vitro* peut être réalisée en amplifiants les séquences codant les domaines VH et VL par polymérisation en chaîne par réaction (PCR) en utilisant des amorces complémentaires des régions CDR3, portant des mutations aléatoires, de manières à ce que des domaines VL et VH amplifiés soient mutés. Ces domaines sont ensuite à nouveau criblés vis à vis de leur capacité à se lier à la cytokine d'intérêt, et les domaines présentant l'affinité la plus importante pour la cytokine sont sélectionnés. L'ADN codant pour les domaines VL et VH est ensuite replacé soit au sein de séquences codant les régions constantes de l'anticorps dont ils sont issus soit au sein de séquences codant les régions constantes d'immunoglobulines quelconques.

Les séquences d'ADN ainsi obtenues, codant pour des anticorps recombinants complets ou pour des fragments de type scFv ou des domaines VH ou VL sont ensuite introduites dans des vecteurs d'expression, et des cellules hôtes, dont des exemples seront fournis ci-après.

D'autres anticorps peuvent être obtenus grâce à un criblage à travers une banque de phages d'expression., préparée en utilisant des ADNc codant pour domaines VL et VH, ces ADNc étant préparés à partir d'ARNm dérivé de lymphocytes humains. Les méthodes de préparation de ces banques sont connues. A titre d'exemple de banque de phage d'expression on peut citer le « Recombinant Phage Antibody System » commercialisé par Pharmacia. Des exemples de réalisation et de criblage de banques de phage d'expression sont fournis dans le brevet US 5,223,409, et dans la demande internationale PCT WO92/20791.

Dans un mode de réalisation, des anticorps monoclonaux humains anti cytokine tels que décrits ci-dessus sont utilisés pour sélectionner des domaines VH et VL ayant des propriétés de liaison aux cytokines similaires. Cette sélection s'effectue par exemple par la méthode de l'« epitope imprinting » la méthode de la sélection guidée ou par les méthodes décrites dans la demande internationale PCT WO93/06213. Les banques d'anticorps utilisées dans cette méthode seront de préférence des banques scFv.

Une fois que les domaines VH et VL sont sélectionnés, ils sont mélangés puis criblés en fonction de leur capacité à se lier à une cytokine sélectionnée. La séquence d'ADN codant les paires de domaines VH et VL peut ensuite être mutée de manière aléatoire, pour augmenter leur affinité vis à vis de la cytokine sélectionnée. Les paires de domaines VH et VL peuvent aussi être mutées dans la région CDR3 selon un procédé analogue à la mutation somatique responsable de la maturation de l'affinité des anticorps durant la réponse immune naturelle. La maturation de l'affinité *in vitro* peut être réalisée selon la méthode décrite ci-dessus, concernant l'ADN d'un lymphocyte B sélectionné pour sa capacité à se lier à une cytokine. Les séquences d'acides aminés des domaines VH et VL sélectionnés peuvent ensuite être comparées aux séquences d'acides aminés correspondantes des lignées germinales car des différences de séquence, dues par exemple au type de banque ayant servi au premier criblage peuvent apparaître. Dans ce cas, il peut être utile de réaliser des mutations « reverses » dans les séquences d'ADN correspondantes pour retrouver la séquence d'acides aminés initiale. De telles mutations reverses peuvent être réalisées par des méthodes classiques en biologie moléculaire, permettant d'introduire des mutations spécifiques, il s'agit par exemple de la mutagenèse en site dirigée.

Après une telle sélection, les acides nucléiques codant les anticorps d'intérêt peuvent être clonés dans des vecteurs d'expression selon des techniques connues. Les acides nucléiques peuvent également subir une étape de manipulation supplémentaire consistant à créer d'autres anticorps par exemple en ajoutant des séquences codant pour des domaines d'immunoglobulines supplémentaires, telles que des régions constantes additionnelles.

Les séquences d'ADN codant les anticorps recombinants ou les fractions d'anticorps, et en particulier les domaines VH et VL peuvent être introduites dans une lignée cellulaire appropriée, comme des cellules de myélome non productrices d'anticorps utilisées pour les hybridomes, de manière à produire les anticorps recombinants sélectionnés pour leur capacité à se lier à une cytokine d'intérêt. Ces séquences d'ADN peuvent également être clonées dans un vecteur d'expression recombinant introduit dans une cellule hôte de mammifère, tel que cela est décrit ci-dessous.

Les hôtes cellulaires préférés sont les cellules CHO, (Chinese Hamster Ovary), les cellules de myélome NS0, les cellules COS, et les cellules SP2. Lorsque des vecteurs d'expression comprenant les séquences codant pour les anticorps, les anticorps recombinants, et les fragments d'anticorps sont introduits dans les cellules hôtes, ces cellules sont cultivées pendant des périodes suffisamment longues pour que les anticorps ou les fragments puissent être produits dans les hôtes cellulaires ou mieux sécrétés dans le milieu de culture dans lequel sont placées les cellules hôtes. Les anticorps sont ensuite récupérés par des techniques standards de purification des protéines.

### Compositions pharmaceutiques et administration.

Des compositions spécifical dans la présente description incluent des compositions pharmaceutiques comprenant des anticorps naturels humains neutralisants anti-cytokine ou tels que décrits ci-dessus, y compris des fragments d'anticorps. Une telle composition comprend en outre au moins un excipient physiologiquement acceptable.

Le terme « excipient physiologiquement acceptable » inclut par exemple les solvants, les milieux de dispersion, les agents anti-bactériens, les agent anti-fongiques ou encore les agents de dilution permettant d'atteindre l'isotonie. Parmi ces excipients conformes à l'invention, on peut citer en particulier l'eau, les tampons phosphate, le dextrose, le glycérol, l'éthanol, et leurs combinaisons. Il est souvent préférable d'ajouter à la composition, des agents isotoniques, tels que des sucres, des polyalcools, par exemple le mannitol ou le sorbitol ou encore le chlorure de sodium. La composition peut en outre comprendre des substances auxiliaires, telles que des émulsifiants, des conservateurs ou des tampons qui vont augmenter l'efficacité ou la durée d'utilisation de ces compositions.

Les compositions ci-dessus peuvent avoir plusieurs formes, et peuvent être par exemple, liquide ou sous forme de doses injectables solides ou semi-solides destinées à être infusées avant injection. D'autres formes sont envisageables, comme par exemple, les suspensions, les dispersions, les comprimés, les poudres, les liposomes ou encore les suppositoires. Les compositions préférées sont sous forme de solutions injectables, similaires à celles qui existent en ce qui concerne la vaccination passive des humains, avec d'autres anticorps que ceux décrits dans la présente demande. Le mode d'administration préféré des compositions est le mode parentéral (par exemple sous cutané ou intramusculaire) ou intraveineux.

Les compositions ci-dessus, doivent être stériles, et stable dans les conditions de fabrication et de stockage habituels. Ces compositions peuvent être des solutions, microémulsions, dispersions, des liposomes ou d'autres structures. La préparation de compositions injectables stériles peut se faire en incorporant le principe actif, c'est à dire l'anticorps ou le fragment d'anticorps, dans une quantité appropriée de solvant et d'autres excipients, tels que décrits ci-dessus, puis à réaliser une stérilisation par filtration.

On peut envisager éventuellement que la composition ci-dessus comprenne un premier type d'anticorps, tels que décrits ci-dessus, neutralisant l'activité d'une cytokine et un deuxième type d'anticorps dirigés par exemple contre des récepteurs ou des cibles connues de ladite cytokine.

Compte tenu du rôle pléiotropique des cytokines, et en particulier de leur rôle dans la réaction inflammatoire et dans la réponse aux l'infections, il peut être avantageux de combiner une composition ci-dessus avec un principe actif supplémentaire, impliqué dans la lutte contre l'inflammation, l'infection, l'asthme, ou encore les rejets de greffes, ou les maladies autoimmunes.

Des exemples d'agents thérapeutiques de lutte contre l'inflammation, qui peuvent être utilisés en combinaison avec les anticorps de l'invention sont :

Le budenoside, les corticostéroides, la cyclosporine, la sulfasalazine les aminosalicylates, la 6-mercaptopurine, l'azathioprine, le métronidazole, les inhibiteurs de la lipoxygénase la mésalamine, l'olsalazine, le balsalazide, les antioxydants, les inhibiteurs de la thromboxane, les antagonistes des récepteurs à l'IL -1, les anticorps monoclonaux anti IL-1β, les anticorps monoclonaux anti-IL-6, les inhibiteurs d'élastase, les récepteur du complément de type 1 sous forme soluble, et autres.

Des exemples d'agents thérapeutiques de lutte contre l'infection, qui peuvent être utilisés en combinaison avec les anticorps de l'invention sont : les antibiotiques, les chélateurs du fer, l'apolipoprotéine 1 reconstituée avec des lipides, des acides hydroxamiques, (agents antibactériens synthétiques).

Les compositions pharmaceutiques ci-dessus peuvent inclure « une quantité pharmaceutique efficace » d'un anticorps ou d'un fragment d'anticorps selon l'invention. Par « quantité pharmaceutique efficace » on entend une quantité efficace, à des dosages et pour une durée suffisante permettant d'aboutir au résultat thérapeutique poursuivi. Cette quantité varie en fonction de facteurs tels que l'état pathologique, le sexe, le poids, l'âge du patient à traiter, et de la capacité de l'anticorps à induire un effet chez le patient. Une telle quantité est entraîne des effets secondaires ou toxiques inférieurs aux bénéfices que peut apporter le traitement par la composition ci-dessus.

La posologie doit être adaptée afin d'obtenir une réponse optimum. Par exemple la composition pharmaceutique peut être administrée en une seule prise ou en plusieurs prises à intervalles de temps réguliers.

### Utilisations

Etant donné la capacité des anticorps contenus dans les compositions selon l'invention à se lier aux cytokines, les anticorps spécifiés dans la présente description peuvent être utilisés pour détecter des cytokines *in vitro,* en utilisant des techniques classiques comme la chromatographie d'affinité, les techniques de type « ELISA » (enzyme linked immunosorbent assays ou encore « RIA » (radioimmunoassay).

On décrit aussi une méthode de détection d'une cytokine dans un échantillon, comprenant une étape de mise en contact de la composition de l'invention avec l'échantillon à analyser et une étape de détection d'un complexe formé entre l'anticorps et une cytokine.

Pour faciliter la détection d'un complexe, l'anticorps peut être marqué par une substance détectable, par exemple, des enzymes comme la peroxydase de raifort, la phosphatase alcaline, la β-galactosidase, ou encore l'acétylcholine estérase, des groupements prosthétiques comme les couples streptavidine/biotine, et avidine/biotine, des molécules fluorescentes, comme l'ombelliférone, la fluorescéine, la rhodamine, la phycoérythrine, ou des molécules radioactives, comme ¹²⁵I, ¹³¹I, ³⁵S, ou ³H.

On décrit aussi une méthode de détection de la présence et de la quantité d'une cytokine dans un échantillon de composition inconnue par dosage par inhibition compétitive. Cette méthode utilise des anticorps tels que spécifiés dans les présente description et une cytokine de référence, marquée.

Selon cette méthode de dosage, l'échantillon biologique, la cytokine de référence marquée, et l'anticorps selon l'invention sont combinés, et la quantité de cytokine de référence liée à l'anticorps est déterminée. La quantité de cytokine de l'échantillon, est inversement proportionnelle à la quantité de cytokine de référence liée à l'anticorps de l'invention.

On décrit également :
- L'utilisation des anticorps anti-VEGF pour la préparation d'une composition pharmaceutique destinée au traitement du cancer.
- L'utilisation des anticorps anti-IFNα pour la préparation d'une composition pharmaceutique destinée au traitement de l'état d'immunosuppression.
- L'utilisation des anticorps anti-IL-4 pour la préparation d'une composition pharmaceutique destinée au traitement de l'allergie.
- L'utilisation des anticorps anti-TNFα pour la préparation d'une composition pharmaceutique destinée au traitement de l'état d'immunosuppression.
- L'utilisation des anticorps anti-TGFβ pour la préparation d'une composition pharmaceutique destinée au traitement de l'infection par un rétrovirus ou au traitement d'un cancer choisi parmi le cancer du colon, le cancer du sein, le cancer de la prostate.

### Méthode de traitement

De manière préférée, la composition d'anticorps spécifiée dans les présente description s'intègre dans le cadre d'une vaccination anti-cytokine en tant que préalable à une vaccination conventionnelle dont le but est de neutraliser ou de bloquer les effets immunotoxiques du stroma, et de permettre le déroulement normal de la réaction immunitaire adaptée contre un agresseur antigénique.

On décrit également une méthode de traitement comprenant au moins les étapes suivantes :
(i) administrer une composition d'anticorps telle que spécifiée dans la présent description à un patient,
(ii) administrer au dit patient une composition immunogène, contenant un antigène ou une combinaison d'antigènes induisant la réponse immunitaire recherchée, à l'encontre dudit antigène ou de ladite combinaison d'antigènes.

Cette méthode en deux étapes permet de rétablir la réponse immunitaire d'un patient, avant que celui-ci ne soit vacciné contre l'agent responsable de la dépression immune.

### Exemple 1: Hétérocomplexe KLH-VEGF humain.

Cet hétérocomplexe est propre à induire principalement chez le vacciné la production d'anticorps qui neutralisent le VEGF humain.

0,58 mg de protéine KLH sont dissous dans 0,5 ml de tampon phosphate 10 mM pH 8,5. A cette solution sont ajoutés 1 mg de VEGF humain dissous dans 1 ml du même tampon.

Le mélange de protéine ainsi obtenu est traité par du glutaraldéhyde à la concentration finale de 0,026 M pendant 30 minutes à température ambiante.

L'excès de glutaraldéhyde est ensuite éliminé par 3 dialyses successives de 2 heures chacune, effectuées dans un boudin de dialyse dont le seuil de coupure est de 3 kDa, à 4 °C, contre 200 ml de tampon phosphate pH 7,6 10 mM.

Le mélange est alors traité par du formaldéhyde à la concentration finale de 33 mM pendant 24 heures. Puis la réaction est bloquée par addition de glycine 0.1 M finale pendant 1 heure à température ambiante. Le mélange est enfin dialysé dans les mêmes conditions que la dialyse effectuée précédemment.

### Exemple 2 : préparation d'un hétérocomplexe KLH-IFNα.

Ce conjugué est propre à induire principalement chez le vacciné la production d'anticorps qui neutralisent l'IFNα humain.

0,625 mg de protéine KLH sont dissous dans 0,6 ml de tampon phosphate 10 mM pH 8,5. A cette solution sont ajoutés 1 mg de protéine IFNα humaine dissous dans 1 ml du même tampon.

Le mélange de protéine ainsi obtenu est traité par du glutaraldéhyde à la concentration finale de 0,026 M pendant 30 minutes à température ambiante.

L'excès de glutaraldéhyde est ensuite éliminé par 3 dialyses successives de 2 heures chacune effectuées dans un boudin de dialyse dont le seuil de coupure est de 3 kDa, à 4 °C, contre 200 ml de tampon phosphate pH 7,610 mM.

Le mélange est alors traité par du formaldéhyde à la concentration finale de 33 mM pendant 48 heures. Puis la réaction est bloquée par addition de glycine 0.1 M finale pendant 1 heure à température ambiante. Le mélange est enfin dialysé dans les mêmes conditions que la dialyse effectuée précédemment.

### Exemple 3 : préparation d'un hétérocomplexe KLH-IL4 humain.

Cet hétérocomplexe est propre à induire principalement chez le vacciné la production d'anticorps qui neutralisent l'IL4 humain.

1 mg de protéine KLH sont dissous dans 1 ml de tampon phosphate 10 mM pH 8,5. A cette solution sont ajoutés 1 mg de protéine IL4 murine dissous dans 1 ml du même tampon.

Le mélange de protéine ainsi obtenu est traité par du glutaraldéhyde à la concentration finale de 0,026 M pendant 30 minutes à température ambiante.

L'excès de glutaraldéhyde est ensuite éliminé par 3 dialyses successives de 2 heures chacune effectuées dans un boudin de dialyse dont le seuil de coupure est de 3 kDa, à 4 °C, contre 200 ml de tampon phosphate pH 7,6 10 mM.

Le mélange est alors traité par du formaldéhyde à la concentration finale de 33 mM pendant 24 heures. Puis la réaction est bloquée par addition de glycine 0.1 M finale pendant 1 heure à température ambiante. Le mélange est enfin dialysé dans les mêmes conditions que la dialyse effectuée précédemment.

### Exemple 4 : préparation d'un hétérocomplexe KLH-TNFα murin.

Ce conjugué propre à induire principalement chez le vacciné la production d'anticorps qui neutralisent le TNFα murin.

0,625 mg de protéine KLH sont dissous dans 0,6 ml de tampon borate 10 mM pH 8,8 150 mM NaCl. A cette solution sont ajoutés 1 mg de protéine IFNα humaine dissous dans 1 ml du même tampon.

Le mélange de protéine ainsi obtenu est traité par du glutaraldéhyde à la concentration finale de 0,026 M pendant 45 minutes à température ambiante.

L'excès de glutaraldéhyde est ensuite éliminé par 3 dialyses successives de 4 heures chacune effectuées dans un boudin de dialyse dont le seuil de coupure est de 3 kDa, à 4 °C, contre 200 ml de tampon phosphate pH 7,6 10 mM 150 mM NaCl.

Le mélange est alors traité par du formaldéhyde à la concentration finale de 33 mM pendant 48 heures. Puis la réaction est bloquée par addition de glycine 0.1 M finale pendant 1 heure à température ambiante. Le mélange est enfin dialysé dans les mêmes conditions que la dialyse effectuée précédemment.

### Exemple 5 test d'activité biologique du VEGF

Des cellules endothéliales du cordon ombilical humain (HUVECs) sont cultivées dans des puits à fond plat d'une plaque de microculture à raison de 3000 cellules par puits. Différentes dilutions d'anticorps sont ensuite déposées sur ces cellules endothéliales. La culture cellulaire est poursuivie à 37 °C en atmosphère humide chargée à 5 % de CO2 pendant 3 jours. 18 heures avant la fin de l'incubation, 0.5 µCi de thymidine tritiée/puits sont ajoutés.

Les anticorps neutralisants empêchent le VEGF murin d'induire la prolifération des cellules endothéliales, alors que les anticorps non neutralisants permettent la prolifération de ces cellules.

### Exemple 6 test d'activité biologique de l'IFNα humain

Des cellules MDBK sont cultivées dans des puits à fond rond d'une plaque de microculture à raison de 350 000 cellules par puits. Différentes dilutions d'IFNα sont ensuite déposées sur les cellules MDBK . Après 20 heures de culture cellulaire réalisée à 37°C en atmosphère humide chargée à 5 % de CO2, les dilutions présentes dans les puits sont enlevés, les cellules lavées, puis 100 µl contenant 100 DL50 (dose léthale 50 %) de virus VSV sont ajoutés. 18 heures après l'addition du virus, l'effet lytique du virus est mesuré.

Les anticorps neutralisants permettent au VSV de lyser les cellules, alors que les anticorps non neutralisants empêchent cette lyse.

### Exemple 7 test d'activité biologique de l'IL 4 humain

Ce test utilise les cellules TF-1, lignée cellulaire humaine dont la croissance est Il4 humaine -dépendante (Kitamura, T. et al., 1989. J.Cell. Physiol 140 : 323-34). Des cellules TF-1 sont cultivées dans des puits à fond rond d'une plaque de microculture à raison de 10 000 cellules par puits. Différentes dilutions sont ensuite déposées sur les cellules TF-1. La culture cellulaire est poursuivie à 37 °C en atmosphère humide chargée à 5 % de CO2 pendant 3 jours. 4 heures avant la fin de l'incubation, 0.5 µCi de thymidine tritiée/puits sont ajoutés.

Les anticorps neutralisants empêchent l'IL4 humain d'induire la prolifération des cellules TF-1, alors que les anticorps non neutralisants permettent la prolifération de ces cellules.

### Exemple 8 test d'activité biologique du TNFα :

Des cellules L929 sont cultivées dans des puits à fond plat d'une plaque de microculture. Différentes dilutions de TNFα sont ensuite déposées sur ces cellules. La culture cellulaire est poursuivie à 37 °C en atmosphère humide chargée à 5 % de CO2 pendant 3 jours. 4 heures avant la fin de l'incubation, 5 mg/ml de MTT sont ajoutés (le MTT est disponible auprès de la société SIGMA Chemical, St. Luois Mo.)

Les anticorps neutralisants empêchent le TNFα d'induire la mort des cellules endothéliales, à l'inverse des anticorps non neutralisants.

### Exemple 9 Comparaison de l'activité biologique des anticorps selon l'invention et des anticorps neutralisants disponibles dans le commerce

### Exemple 9.1 Production d'un anticorps polyclonal anti-interféron α (hu) chez l'homme

Un groupe d'individus volontaires a été immunisé par administration par voie intra musculaire d'interféron α humain inactivé par un traitement chimique à base de diméthylformamide. Chaque individu volontaire à subi une injection de 70 µg d'IFNα inactivé à J0, J7, J14, J21 et J42.

La courbe de production des anticorps anti-interféron α a été déterminée en fonction du temps. Au pic de production, (lorsque le taux d'immunoglobulines d'isotype IgG anti-IFNα généré était environ de 9.5 µg/ml), 20 ml de sang ont été prélevés chez chacun des vaccinés et un pool de sérums constitué. L'activité anti-interféron α du pool a été déterminée par ELISA et son titre trouvé égal à 128 000 (inverse de la dilution donnant une DO = 0.300)

La fraction IgG a été isolée d'une partie du pool de sérum par précipitation par le (NH₄)₂ SO₄ à 35% de saturation. Après dialyse, la solution de IgG contenant les anticorps IgG neutralisant l'activité de l'IFNα obtenu avec un rendement de 80% a présenté une activité à l'ELISA de 512 000.

Le pouvoir neutralisant (quantité d'anticorps permettant d'inhiber de 50 % l'activité de 1 UI d'interferon alpha humain) du sérum entier anti-interféron α et de l'IgG en dérivant s'est établi à 0,004 µg et 0,001 µg, respectivement.

**Tableau 1**

| | **Ac de l'exemple 9.1** | Ac polyclonal de lapin (1) | Ac polyclonal de mouton (2) | Ac monoclonal de souris (3) |
|---|---|---|---|---|
| IFNα humain (1 UI) | **0,04** µ**g** | 0,01 µg | 0,0005 µg | 0,8 µg |

Le tableau 1 établit une comparaison entre le pouvoir neutralisant de l'anticorps polyclonal anti-interféron α produit ci-dessus avec le pouvoir neutralisant d'anticorps du commerce. Le pouvoir neutralisant obtenu avec l'anticorps produit ci-dessus est environ 200 fois supérieur au titre obtenu avec un anticorps monoclonal de souris.
(1) Anticorps polyclonal de lapin dirigé contre l'IFNα, disponible auprès de la société PBL Biomedical Pharmaceutical sous le n° de référence 31100-1.
(2) Anticorps polyclonal de mouton dirigé contre l'IFNα, disponible auprès de la société PBL Biomédical Pharmaceutical sous le n° de référence 31130-1.
(3) Anticorps monoclonal de souris dirigé contre l'IFNα, disponible auprès de la société PBL Biomedical Pharmaceutical sous le n° de référence 21105-1.

### Exemple 9.2 : Production d'un anticorps polyclonal anti-IL-4 chez l'homme

Un sujet volontaire a été immunisé par administration intramusculaire d'un conjugué de KLH IL-4 humain.

Le sujet à subi une injection de 120 µg d'IL-4 humain inactivé chimiquement par du diméthylformamide à J0, J21, et J60.

La courbe de production des anticorps anti-IL-4 a été déterminée en fonction du temps. Au pic de synthèse des anticorps (lorsque le taux d'immunoglobulines d'isotype IgG anti-IL4 généré était environ de 12 µg/ml) un prélèvement de sang a été effectué et le sérum anti-IL-4 recueilli. La mesure par ELISA de l'activité du sérum a donné un titre de 512 000 (inverse de la dilution du sérum donnant une valeur en ELISA de 0,300. Après séparation de la fraction IgG contenant les anticorps anti-IL-4, la fraction a été digérée par la pepsine et le fragment anticorps F_{(ab')2} recueilli avec un rendement de 60%. La détermination de l'activité en ELISA a fourni une valeur de 310 000.

Le pouvoir neutralisant (quantité d'anticorps permettant d'inhiber de 50 % l'activité de 0,5 ng d'interleukine-4) du sérum anti-IL-4 entier s'est établi à 0,02 µg et pour la fraction F_{(ab')2} à 0,1 µg.

**Tableau 2**

| | **Ac de l'exemple 9.2** | Ac polyclonal de chèvre (1) | Ac polyclonal de lapin (2) | Ac monoclonal de souris (3) |
|---|---|---|---|---|
| IL-4 humain (0,5 ng) | **0,02** µ**g** | 0,08 µg | 0,01 µg | 1 µg |

Le tableau 2 établit une comparaison entre le pouvoir neutralisant de l'anticorps polyclonal anti-IL-4 produit ci-dessus avec le pouvoir neutralisant des anticorps du commerce. Le titre obtenu avec l'anticorps produit ci-dessus est environ 50 fois supérieur au titre obtenu avec un anticorps monoclonal de souris.
(1) Anticorps polyclonal de chèvre dirigé contre IL-4, disponible auprès de la société R&D sous le n° de référence AF-204-NA.
(2) Anticorps polyclonal de lapin dirigé contre IL-4 disponible auprès de la société Peprotech sous le n° de référence 500-P24.
(3) Anticorps monoclonal de souris dirigé contre IL-4 disponible auprès de la société R&D sous le n° de référence MAB204.

### Exemple 9.3 : Production d'un anticorps polyclonal anti-VEGF murin chez la souris

Une méthode semblable à celle décrite pour les exemples 7.1 et 7.2 est utilisée. Un groupe de 20 souris a été immunisé par administration d'un hétérocomplexe de KLH-VEGF murin. Chaque souris à subi une injection de 20 µg de VEGF inactivé chimiquement par du diméthylformamide à J0, J7, J14, J21 et J42.

En se rapportant à la courbe de production des anticorps anti-VEGF, un prélèvement de sang a été effectué chez toutes les souris au pic de réponse et un pool de sérum anti-VEGF constitué. La fraction IgG a été isolée par précipitation par le (NH₄)₂ SO₄ à 35% de saturation. L'activité ELISA a été déterminée et est égale à 256 000 et l'activité neutralisante de la fraction IgG (quantité d'anticorps permettant d'inhiber de 50 % l'activité de 0,5 ng de VEGF murin) à 0,012 µg.

**Tableau 3**

| | **Ac de l'exemple 9.3** | Ac polyclonal de chèvre (1) | Ac polyclonal de lapin (2) | Ac monoclonal De souris (3) |
|---|---|---|---|---|
| VEGF (0,5 ng) | **0,012 µg** | 0,001 µg | 0,007 µg | 0,003 µg |

Le tableau 3 établit une comparaison entre le pouvoir neutralisant de l'anticorps polyclonal anti-VEGF murin produit ci-dessus avec le pouvoir neutralisant des anticorps anti-VEGF du commerce.
(1) Anticorps polyclonal de chèvre dirigé contre le VEGF, disponible auprès de la société R&D sous le n° de référence AF-293-NA.
(2) Anticorps polyclonal de lapin dirigé contre le VEGF disponible auprès de la société Peprotech sous le n° de référence 500-P10.
(3) Anticorps monoclonal de souris dirigé contre le VEGF disponible auprès de la société R&D sous le n° de référence MAB293.

### Exemple 9.4: Production d'un anticorps monoclonal recombinant dirigé contre l'IFNα humain

### Matériel :

Les cellules mononucléaires du sang humain périphérique (PBMC) proviennent d'un sujet immunisé avec de l'IFNα humain inactivé chimiquement.

Le virus d'Epstein Barr (EBV) a été préparé à partir d'un surnageant de culture de la lignée cellulaire B95-8 et utilisé à la concentration 10⁵ TD₅₀/ml.

### Culture :

Les PBMCs ont été purifiés par gradient de Ficoll puis infectées par le virus. Les cellules transformées par l'EBV sont cultivées en plaque de 96 puits à fond rond à la concentration de 5 x 10³-10⁴ /ml avec du milieu RPMI 1640 (Gibco BRL, Life Technologies) additionné de 20% de SVF (Gibco BRL, Life Technologies). Le milieu est changé tous les 4 jours. Après 4 à 6 semaines de culture, les cellules sont transférées dans des plaques 24 puits puis finalement dans des plaques 6 puits. Le surnageant de culture est ensuite analysé par ELISA.

### Sélection des clones producteurs d'anticorps monoclonal anti-IFNa.

Les clones produisant des anticorps anti-IFNα sont sélectionnés et mis en culture de masse. Un anticorps monoclonal a été purifié à partir d'un surnageant de culture par adsorption sur une colonne de sepharase Protéine A. L'anticorps monoclonal produit a été purifié par adsorption sur une colonne de sepharose Protéine A. La solution d'anticorps monoclonal anti- l'IFNα purifié présente une teneur en anticorps monoclonal de 1 mg/ml et son activité neutralisante (pouvoir d'inhibition de 50% de l'activité d'1 UI d'IFNα) ou ND50 égale à 0,7 µg.

Ces clones produisent des anticorps représentent un matériel cellulaire à haut rendement pour la production d'un anticorps monoclonal humain anti- l'IFNα (hu) à haute affinité en utilisant la technologie du phage display connue de l'homme de l'art et décrite dans la présente demande.

**Tableau 4**

| | **Ac de l'exemple 9.4** | Ac polyclonal de lapin (1) | Ac polyclonal de mouton (2) | Ac monoclonal de souris (3) |
|---|---|---|---|---|
| IFNα humain (1 UI) | **0,7** µ**g** | 0,01 µg | 0,0005 µg | 0,8 µg |

Le tableau 4 établit une comparaison entre le pouvoir neutralisant de l'anticorps monoclonal IFNα produit ci-dessus avec le pouvoir neutralisant des anticorps du commerce. Le titre obtenu avec l'anticorps produit ci-dessus est environ 50 fois supérieur au titre obtenu avec un anticorps monoclonal de souris.
(1) Anticorps polyclonal de lapin dirigé contre l'IFNα, disponible auprès de la société PBL Biomedical Pharmaceutical sous le n° de référence 31100-1.
(2) Anticorps polyclonal de mouton dirigé contre l'IFNα, disponible auprès de la société PBL Biomedical Pharmaceutical sous le n° de référence 31130-1.
(3) Anticorps monoclonal de souris dirigé contre l'IFNα, disponible auprès de la société PBL Biomédical Pharmaceutical sous le n° de référence 21105-1.

### Exemple 10 : Détermination du pourcentage de cytokine fixée sur la protéine porteuse (KLH) par ELISA double sandwich

Le pourcentage de cytokine fixée sur la protéine porteuse (KLH) a été déterminé par un ELISA double sandwich, à l'aide d'un anticorps de capture dirigé spécifiquement contre la protéine porteuse.

100 µl d'anticorps polyclonaux de chevaux dirigés contre le KLH (1 mg/ml) dilués dans un tampon phosphate 10 mM pH 7,3 NaCl 150 mM (PBS) sont fixés dans des puits d'une plaque de microtitration (high-binding; Costar) pendant 2 heures à 37°C. Après 3 lavages dans du PBS/0.1% Tween 20 (PBST), les puits sont saturés avec du PBS contenant 2 % de SVF. Après 1h30 de saturation, les puits sont lavés 3 fois avec du PBST, puis des dilutions de 2 en 2 d'hétérocomplexe (10, 5, 2.5, 1.25, 0.625, 0.312 et 0.156 µg/ml), réalisées en doublon, sont ajoutées dans les puits (100 µl/puits). Après 2 heures d'incubation, les puits sont lavés 3 fois avec le PBST. Le tween, agent dissociant, présent dans le tampon de lavage, permet d'éliminer toutes molécules non fixées de manière covalente sur le KLH qui lui est fixé spécifiquement sur l'anticorps de capture. Ensuite les deux dilutions d'hétérocomplexe sont traitées de 2 façons différentes
a) la première série est incubée avec un anticorps dirigé contre le KLH
b) la deuxième série est incubée avec un anticorps dirigé contre la cytokine.

Après 1 h30 d'incubation à 37 °C, les puits sont lavés comme précédemment puis incubés avec un anticorps secondaire couplé à la peroxydase, dirigé contre l'espèce d'origine du premier anticorps. Après 1h30 d'incubation à 37 °C, les puits sont à nouveau lavés. Ensuite l'addition du substrat de la peroxydase, O-PhénylèneDiamine, (OPD), permet de révéler la présence du KLH fixé par l'anticorps de capture et des cytokines fixées de manière covalente sur le KLH.

La quantité de KLH fixé par l'anticorps de capture puis la quantité de molécules de cytokine fixée sur le KLH de manière covalente sont calculées à l'aide de courbes d'étalonnage faites par ELISA.

Le pourcentage de cytokine fixée de manière covalente au KLH est alors déterminé.

### Références bibliographigues

Eastcott JW, Holmberg CJ, Dewhirst FE, Esch TR, Smith DJ, Taubman MA. (2001) Oligonucleotide containing CpG motifs enhances immune response to mucosally or systemically administered tetanus toxoid. Vaccine. 2001 Feb 8;19(13-14):1636-42.
Gallichan WS, Woolstencroft RN, Guarasci T, McCluskie MJ, Davis HL, Rosenthal KL. (2001) Intranasal immunization with CpG oligodeoxynucleotides as an adjuvant dramatically increases IgA and protection against herpes simplex virus-2 in the genital tract. J Immunol. 2001 Mar 1;166(5):3451-7.
Kitamura T, Tange T, Terasawa T, Chiba S, Kuwaki T, Miyagawa K, Piao YF, Miyazono K, Urabe A, Takaku F.. (1989) Establishment and characterization of a unique human cell line that proliferates dependently on GM-CSF, IL-3, or erythropoietin. J Cell Physiol Aug;140(2):323-34.
McCluskie MJ, Weeratna RD, Davis HL (2000) Intranasal immunization of mice with CpG DNA induces strong systemic and mucosal responses that are influenced by other mucosal adjuvants and antigen distribution. Mol Med. Oct;6(10):867-77.
Rippmann JF, Klein M, Hoischen C, Brocks B, Rettig WJ, Gumpert J, Pfizenmaier K, Mattes R, Moosmayer D. (1998) Procaryotic expression of single-chain variable-fragment (scFv) antibodies: secretion in L-form cells of Proteus mirabilis leads to active product and overcomes the limitations of periplasmic expression in Escherichia coli. Appl Environ Microbiol. 1998 Dec;64(12):4862-9.

## Revendications

1. Composition vaccinale **caractérisée en ce qu'**elle comprend, à titre de principe actif, un produit immunogène stable comprenant des hétérocomplexes immunogènes protéiques constitués d'associations entre (i) des molécules de protéines antigéniques inactivées, respectivement choisies parmi le l'IFNα, l'IL-4, et le TNFα, et (ii) des molécules protéiques porteuses dans lesquels moins de 40 pour cent des protéines antigéniques inactivées (i) sont liées avec les molécules protéiques porteuses (ii) par une liaison covalente.

2. Composition vaccinale selon la revendication 1, **caractérisée en ce qu'**elle comprend en outre des oligodésoxynucléotides CpG à titre d'adjuvant.

3. Composition vaccinale **caractérisée en ce qu'**elle comprend, à titre de principe actif, un produit immunogène stable comprenant des hétérocomplexes immunogènes protéiques constitués d'associations entre (i) des molécules de protéines antigéniques inactivées, respectivement choisies parmi le l'IFNα, l'IL-4, et le TNFα, et (ii) des molécules protéiques porteuses dans lesquels moins de 40 pour cent des protéines antigéniques inactivées (i) sont liées avec les molécules protéiques porteuses (ii) par une liaison covalente pour l'utilisation en tant que médicament.

## Claims

1. Vaccine composition comprising, as the active ingredient, a stable immunogenic product comprising protein immunogenic heterocomplexes consisting of combinations of (i) molecules of antigenic proteins, respectively selected from IFNα, IL-4 and TNFα and (ii) protein carrier molecules in which less than 40% of the antigenic proteins (i) are linked with protein carrier molecules (ii) through a covalent bond.

2. Vaccine composition according to claim 1, **characterized in that** it further comprises CpG oliogodesoxynucleotides as an adjuvant.

3. Vaccine composition comprising, as the active ingredient, a stable immunogenic product comprising protein immunogenic heterocomplexes consisting of combinations of (i) molecules of antigenic proteins, respectively selected from IFNα, IL-4 and TNFα and (ii) protein carrier molecules in which less than 40% of the antigenic proteins (i) are linked with protein carrier molecules (ii) through a covalent bond, for use as a medicament.

## Patentansprüche

1. Impfzusammensetzung, **dadurch gekennzeichnet, dass** sie als Wirkstoff ein stabiles immunogenes Produkt umfasst, das immunogene Heteroproteinkomplexe umfasst, die aus Assoziationen aufgebaut sind zwischen (i) inaktivierten antigenen Proteinmolekülen, die jeweils aus IFNα, IL-4 und TNFα ausgewählt sind, und (ii) Trägerproteinmolekülen, wobei weniger als 40 Prozent der inaktivierten antigenen Proteine (i) durch eine kovalente Bindung mit den Trägerproteinmolekülen (ii) verknüpft sind.

2. Impfzusammensetzung gemäß Anspruch 1, **dadurch gekennzeichnet, dass** sie weiterhin CpG-Oligodesoxynucleotide als Adjuvans umfasst.

3. Impfzusammensetzung, **dadurch gekennzeichnet, dass** sie als Wirkstoff ein stabiles immunogenes Produkt umfasst, das immunogene Heteroproteinkomplexe umfasst, die aus Assoziationen aufgebaut sind zwischen (i) inaktivierten antigenen Proteinmolekülen, die jeweils aus IFNα, IL-4 und TNFα ausgewählt sind, und (ii) Trägerproteinmolekülen, wobei weniger als 40 Prozent der inaktivierten antigenen Proteine (i) durch eine kovalente Bindung mit den Trägerproteinmolekülen (ii) verknüpft sind, zur Verwendung als Medikament.
